(19) Europäisches Patentamt
European Patent Office
Office européen des brevets

(11) EP 1 422 213 A1

(12) **EUROPEAN PATENT APPLICATION**
published in accordance with Art. 158(3) EPC

(43) Date of publication:
**26.05.2004 Bulletin 2004/22**

(21) Application number: **02767862.2**

(22) Date of filing: **27.08.2002**

(51) Int Cl.7: **C07C 49/173**, C07C 45/26, C07C 45/45, C07C 45/65, C07D 319/12, C12P 7/18

(86) International application number:
**PCT/JP2002/008624**

(87) International publication number:
**WO 2003/018523 (06.03.2003 Gazette 2003/10)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **27.08.2001 JP 2001255847**

(71) Applicant: **KANEKA CORPORATION**
**Osaka-shi, Osaka 530-8288 (JP)**

(72) Inventors:
- **TAOKA, Naoaki**
**KANEKA CORPORATION Takasago Plant**
**Takasago-shi, Hyogo 676-0027 (JP)**
- **NISHIYAMA, Tozo**
**KANEKA CORPORATION Takasago Plant**
**Takasago-shi, Hyogo 676-0027 (JP)**
- **KISAKI, Noriyuki**
**KANEKA CORPORATION Takasago Plant**
**Takasago-shi, Hyogo 676-0027 (JP)**
- **YASOHARA, Yoshihiko**
**KANEKA CORPORATION Takasago**
**Takasago-shi, Hyogo 676-0027 (JP)**

(74) Representative: **VOSSIUS & PARTNER**
**Siebertstrasse 4**
**81675 München (DE)**

# (54) PROCESS FOR PRODUCING OPTICALLY ACTIVE HALOPROPANEDIOL DERIVATIVE

(57) The present invention provides a method capable of simply producing optically active halopropanediol derivatives useful as pharmaceutical intermediates from inexpensive raw materials.

Monohalohydroxyacetone derivatives can be produced by reacting propargyl alcohol derivatives available at low cost and a hypohalogenous acid to convert the propargyl alcohol derivatives to di- or tri-halohydroxyacetone derivatives, and then hydrogenating the derivatives in the present of a transition metal catalyst. Also, optically active halopropanediol derivatives can be produced by stereoselectively reducing the halohydroxyacetone derivatives with an enzyme source having the ability to stereoselectively reduce the carbonyl groups of the halohydroxyacetone derivatives.

EP 1 422 213 A1

## Description

Technical Field

The present invention relates to a method for producing optically active halopropanediol derivatives, particularly optically active 3-chloro-1,2-propanediol, useful as pharmaceutical intermediates. More specifically, the present invention relates to a method for producing optically active halopropanediol derivatives, particularly optically active 3-chloro-1,2-propanediol, the method comprising synthesizing halohydroxyacetone derivatives from inexpensive raw materials by a simple method, and then stereoselectively reducing the halohydroxyacetone derivatives with an enzyme source having the ability to stereoselectively reduce the carbonyl groups thereof.

Background Art

Examples of a conventional known method for producing optically active halopropanediol derivatives include the following.

1) A method in which only one of optical isomers of racemic 3-chloro-1,2-propanediol is decomposed by assimilation with microorganisms to leave (S) or (R)-3-chloro-1,2-propanediol (Chemistry and Chemical Industry, 1997, Vol. 71, pp. 482-489).
2) A method in which racemic 3-chloro-1,2-propanediol is converted to (R)-3-chloro-1,2-propanediol and (S)-thioglycerin by treatment with microorganisms in the presence of a thiol compound, and then (R)-3-chloro-1,2-propanediol is isolated and purified (Japanese Unexamined Patent Application Publication Nos. 62-122596 and 62-122597).
3) A method in which 1-acetoxy-3-chloro-2-propanone is stereoselectively reduced with microorganisms and then deacetylated with lipase to produce optically active 3-halo-1,2-propanediol (Japanese Unexamined Patent Application Publication No. 11-103878).
4) A method in which racemic epichlorohydrin is converted to (S)-epichlorohydrin (yield 44%, optical purity 96%ee) and (R)-3-chloro-1,2-propanediol (yield 50%, optical purity 96%ee) by asymmetric ring opening in the presence of a (R,R) Jacobsen catalyst, and then (R)-3-chloro-1,2-propanediol is isolated and purified (WO00/009463).

However, the method 1) is an optical resolution method and thus has a theoretical yield of 50% or less, and the method has low productivity due to a low feed ratio of the raw material. The method 2) secondarily produces a large amount of waste such as a sulfur-containing compound, or the like. The method 3) is complicated due to unavailability of a substrate, and the need for deacylation with lipase. The method 4) requires an expensive asymmetric catalyst and produces optically active 3-chloro-1,2-propanediol with low optical purity. In this way, any one of these methods has a great problem in industrial applications.

On the other hand, a method comprising stereoselectively reducing the carbonyl group of a halohydroxyacetone derivative, which is a prochiral compound, with microorganisms to obtain optically active halopropanediol has not been reported because of difficulties in synthesizing, and isolating and purifying the halohydroxyacetone derivative used as the substrate.

Known methods for producing halohydroxyacetone derivatives include the following.

5) A method in which $\alpha$-chloropyruvic acid is treated with methyl orthoformate/methanol in the presence of sulfuric acid, and then reduced with lithium aluminum hydride to produce 3-chloro-2,2-dimethyloxy-1-propanol, which is then treated with an acid to obtain an aqueous solution of 3-chloro-1-hydroxy-2-propanone (J. Org. Chem., 1982, 47, 2355-2358).
6) A method in which 2-chloro-2-propene-1-ol is treated with N-bromosuccinimide (NBS) to obtain 3-bromo-1-hydroxy-2-propanone (Tetrahedron Lett., 1993, 34, 1733-1736).
7) A method in which chloropropargyl alcohol is treated with formaldehyde in the presence of potassium carbonate, and then the resultant product is purified by distillation to obtain 1-chloro-2,3-methylenedioxypropene which is then treated with an acid to obtain 3-chloro-1-hydroxy-2-propanone (Justus Liebigs Ann. Chem., 1969, 724, 128-136).

However, the method 5) must use an expensive reducing agent and requires a large number of steps and is thus complicated. The method 6) uses a starting raw material which is not easily available and requires expensive NBS. The method 7) uses an expensive starting raw material and comprises complicated operations. Therefore, these methods are industrial production methods having needs to be improved.

Disclosure of the Invention

In consideration of the above situation, an object of the present invention is to provide a method capable of

simply producing optically active halopropanediol derivatives, particularly optically active 3-chloro-1,2-propanediol, useful as pharmaceutical intermediates from easily-available inexpensive raw materials.

As a result of intensive research in consideration of the above-mentioned situation, the inventors found a method for simply producing optically active halopropanediol derivatives, the method comprising synthesizing halohydroxyacetone derivatives from propargyl alcohol derivatives easily available at low cost by a simple method, and then stereoselectively reducing the halohydroxyacetone derivatives with an enzyme source having the ability to stereoselectively reduce the carbonyl groups thereof, leading to the achievement of the present invention.

Namely, the present invention relates to dihalohydroxyacetone derivatives represented by formula (9) or formula (10):

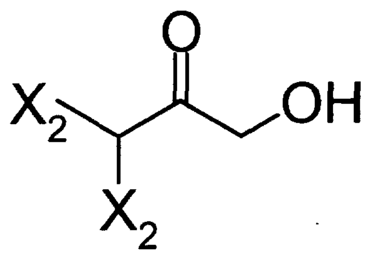

(9)

(wherein $X_2$ represents a halogen atom);

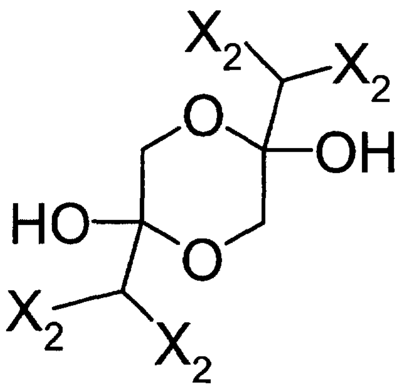

(10)

(wherein $X_2$ is the same as the above).

The present invention also relates to a method for producing di- or tri-halohydroxyacetone derivatives represented by formula (3) or formula (4):

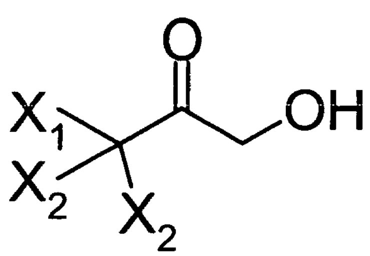

(3)

(wherein $X_1$ represents a hydrogen atom or a halogen atom, and $X_2$ represents a halogen atom);

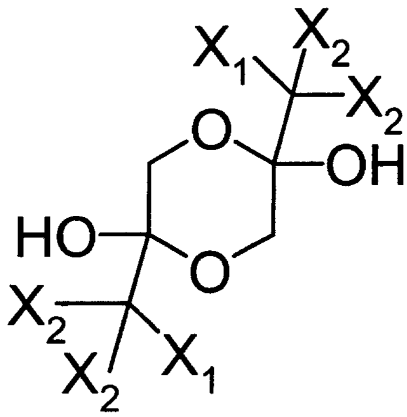

(4)

(wherein $X_1$ and $X_2$ are the same as the above), the method comprising reacting propargyl alcohol derivatives represented by formula (1) with hypohalogenous acid represented by formula (2):

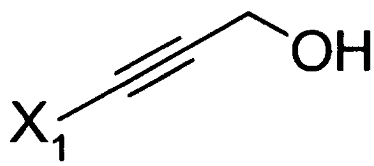

(1)

(wherein $X_1$ represents a hydrogen atom or a halogen atom);

$$X_2OH \quad (2)$$

(wherein $X_2$ represents a halogen atom).

Furthermore, the present invention relates to a method for producing monohalohydroxyacetone derivatives represented by formula (5) or formula (6):

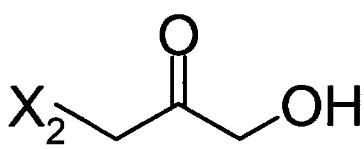

(5)

(wherein $X_2$ represents a halogen atom);

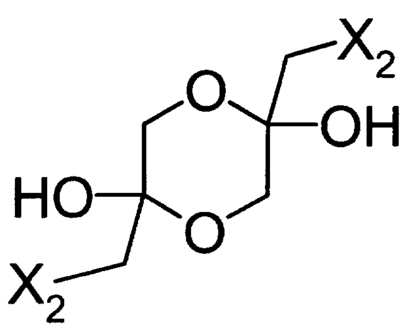

(6)

(wherein $X_2$ is the same as the above), the method comprising hydrogenating di- or tri-halohydroxyacetone derivatives

represented by formula (3) or formula (4) in a solvent in the presence of a transition metal catalyst:

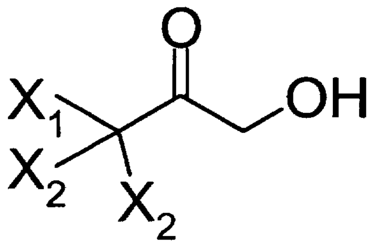

(3)

(wherein $X_1$ represents a hydrogen atom or a halogen atom,
and $X_2$ represents a halogen atom);

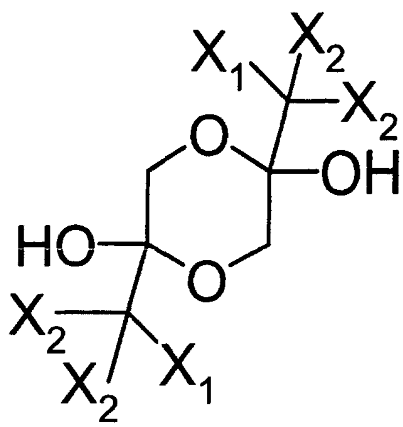

(4)

(wherein $X_1$ and $X_2$ are the same as the above).

Furthermore, the present invention relates to a method for producing optically active halopropanediol derivatives represented by formula (7) or formula (8):

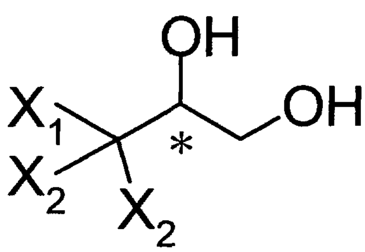

(7)

(wherein $X_1$ represents a hydrogen atom or a halogen atom, $X_2$ represents a halogen atom, and * represents an asymmetric carbon atom);

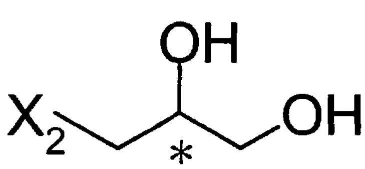

(8)

(wherein $X_2$ and * are the same as the above), the method comprising stereoselectively reducing halohydroxyacetone

derivatives represented by any one of formulae (3), (4), (5), and (6) with an enzyme source having the ability to stereoselectively reducing the carbonyl groups of di- or tri-halohydroxyacetone derivatives represented by formula (3) or (4), or monohalohydroxyacetone derivatives represented by formula (5) or (6):

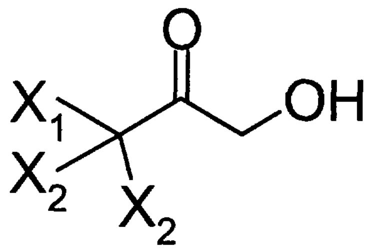

(3)

(wherein $X_1$ represents a hydrogen atom or a halogen atom, and $X_2$ represents a halogen atom);

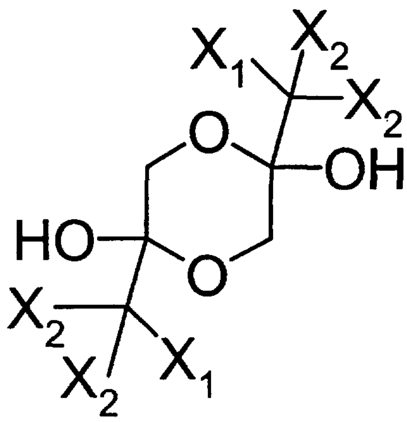

(4)

(wherein $X_1$ and $X_2$ are the same as the above);

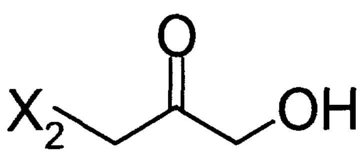

(5)

(wherein $X_2$ is the same as the above);

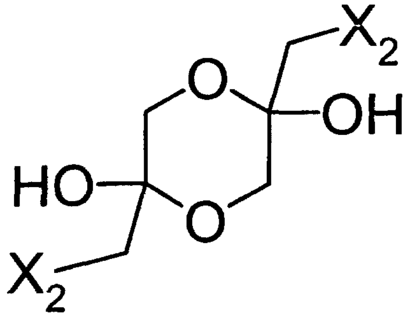

(6)

(wherein $X_2$ is the same as the above).

Furthermore, the present invention relates to a method for isolating and purifying 2,5-dichloromethyl-2,5-dihydroxy-1,4-dioxane, the method comprising removing impurities from 2,5-dichloromethyl-2,5-dihydroxy-1,4-dioxane

containing the impurities and represented by formula (11) by using an organic solvent to obtain as crystals the compound represented by formula (11):

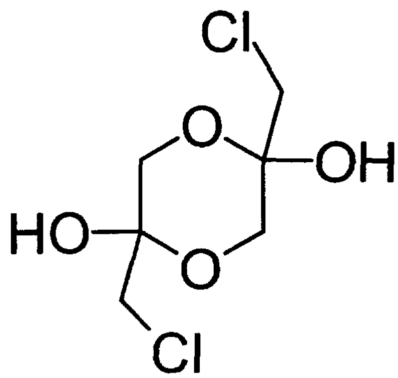

(11)

Detailed Disclosure of the Invention

The present invention will be described in detail below.

In each of formulae (1), (3), (4) and (7), $X_1$ represents a hydrogen atom or a halogen atom such as a fluorine atom, a chlorine atom, a bromine atom, an iodine atom, or the like, and preferably a hydrogen atom. In each of formulae (2) to (10), $X_2$ represents a halogen atom such as a fluorine atom, a chlorine atom, a bromine atom, an iodine atom, or the like, and preferably a chlorine atom. In each of formulae (7) and (8), * represents an asymmetric carbon atom.

Also, dihalohydroxyacetone derivatives represented by formula (9) or (10) are novel compounds which have been not reported in documents and which were found to be useful in producing optically active halopropanediol derivatives by the inventors.

As the propargyl alcohol derivatives represented by formula (1), for example, propargyl alcohol which is industrially available at low cost can be used, or it can be converted to halopropargyl alcohol by halogenation with a halogenating agent and used (Bull. Chem. Soc. Jap., 1972, 45, 2611-2615).

Next, the method for producing optically active halopropanediol derivatives of the present invention will be described.

First, the method for producing di- or tri-halohydroxyacetone derivatives of the present invention will be described.

A propargyl alcohol derivative represented by formula (1) is reacted with a hypohalogenous acid represented by formula (2) to produce a di- or tri-halohydroxyacetone derivative represented by formula (3) or (4).

Examples of a hypohalogenous acid include hypochlorous acid, hypobromous acid, hypoiodous acid, and the like, and hypochlorous acid is preferably used. As the hypohalogenous acid, a commercially available hypohalogenous acid may be used, or the hypohalogenous acid may be prepared by, for example, mixing a sodium hypochlorite solution and an acid or contact between chlorine and water. In regard to the amount of the hypohalogenous acid used, the molar ratio of the hypohalogenous acid to the propargyl alcohol derivative used is preferably 1:1 to 1:5, and more preferably 1:1 to 1:3.

Examples of a reaction solvent usable in the reaction include hydrocarbon solvents such as hexane, heptane, benzene, toluene, and the like; ether solvents such as diethyl ether, tetrahydrofuran, 1,4-dioxane, tert-butyl methyl ether, dimethoxyethane, and the like; halogenated solvents such as methylene chloride, chloroform, 1,1,1-trichloroehtane, and the like; nitrogen-containing solvents such as dimethylformamide, acetamide, formamide, acetonitrile, propionitrile, and the like; aprotic solvents such as dimethylsulfoxide, N-methylpyrrolidone, hexamethylphosphoric triamide, and the like; alcohol solvents such as methanol, ethanol, n-propanol, n-butanol, and the like; and water. Water is preferably used. These reaction solvents may be used individually or in a combination of at least two solvents.

The reaction temperature of the reaction is preferably - 20 to 80°C, and more preferably 0 to 40°C. The reaction time of the reaction is preferably 1 hour to 72 hours, and more preferably 1 hour to 10 hours.

The di- or tri-halohydroxyacetone derivative obtained by the reaction can be purified by a conventional method. For example, 1,1-dichloro-3-hydroxyacetone can be extracted from the reaction mixture with an organic solvent, and then purified by a silica gel column or the like. Alternatively, the reaction mixture may be concentrated in the solvent and then stirred under cooling to convert 1,1-dichloro-3-hydroxyacetone into 2,5-dihydroxy-2,5-di(dichloromethyl)-1,4-dioxane by dimerization. The resulting dimmer can be isolated and purified as a crystal compound.

Next, the method for producing a monohalohydroxyacetone derivative of the present invention will be described below.

The di- or tri-halohydroxyacetone derivative produced by the above-described production method and represented by formula (3) or (4) is hydrogenated in a solvent in the presence of a transition metal catalyst to produce the monohalohydroxyacetone derivative represented by formula (5) or (6).

As the transmission metal catalyst, a platinum catalyst, a rhodium catalyst, a palladium catalyst, a nickel catalyst, a ruthenium catalyst, an iridium catalyst, a rhenium catalyst, or the like can be used. For example, such a transition metal catalyst comprises a metal such as platinum, rhodium, palladium, nickel, ruthenium, iridium, rhenium, or the like; or an alloy, a chloride, a bromide, an iodide, a nitrate, a sulfate, a phosphate, an oxide, a sulfide, a boride, a hydroxide, a cyanide, an acetylacetonate, an acetate, or a trifluoroacetate of the metal.

Concrete examples of the catalyst include platinum metal, platinum black, platinum (II) acetylacetonate, platinum (II) bis(benzonitrile) dichloride, platinum (II) bromide, platinum (IV) bromide, platinum (II) chloride, platinum (IV) chloride, platinum (II) cyanide, platinum (II) iodide, platinum (IV) oxide, platinum (IV) oxide hydrate, platinum rhodium alloys, platinum palladium alloys, platinum iridium alloys, platinum (IV) sulfide; rhodium metal, rhodium black, rhodium (II) acetate, rhodium (II) acetylacetonate, rhodium (II) bromide hydrate, rhodium (III) chloride, rhodium (III) chloride hydrate, rhodium (II) hexafluorobutanoate, rhodium (II) hexanoate, rhodium (III) iodide hydrate, rhodium (III) nitrate, rhodium (III) oxide, rhodium (III) oxide hydrate, rhodium (III) phosphate, rhodium (III) sulfate, rhodium (II) trifluoroacetate; palladium metal, palladium black, palladium (II) acetate, palladium (II) acetylacetonate, palladium(II) bis(benzonitrile) dichloride, palladium (II) bromide, palladium (II) chloride, palladium (II) cyanide, palladium (II) hydroxide, palladium (II) iodide, palladium (II) nitrate, palladium (II) nitrate hydrate, palladium (II) oxide, palladium (II) oxide hydrate, palladium (II) propionate, palladium (II) sulfate, palladium (II) sulfide, palladium (II) trifluoroacetate; nickel metal, Raney nickel, nickel boride, nickel (II) oxide; ruthenium metal, ruthenium black, ruthenium (III) acetylacetonate, ruthenium (III) bromide, ruthenium (III) bromide hydrate, ruthenium (III) chloride, ruthenium (III) chloride hydrate, ruthenium (III) iodide, ruthenium (III) nitrosyl chloride hydrate, ruthenium (III) nitrosylnitrate, ruthenium (IV) oxide, ruthenium (IV) oxide hydrate; iridium metal, iridium (III) acetylacetonate, iridium (III) bromide hydrate, iridium (III) chloride, iridium (III) chloride hydrochloride, iridium (IV) chloride hydrate, iridium (IV) oxide, iridium (IV) oxide hydrate; rhenium metal, rhenium (III) chloride, rhenium (V) chloride, rhenium (IV) fluoride, rhenium (IV) oxide, rhenium (VI) oxide, rhenium (VII) oxide, rhenium (VII) sulfide, and the like. Preferably, platinum oxide, palladium oxide, platinum black, palladium black, Raney nickel, nickel boride, and the like.

Any one of these catalysts is more preferably dispersed in a powder support from the viewpoint of catalytic activity, reproducibility, preservation stability, operationality, ease of recycling, and the like. Examples of the powder support include carbon, alumina, silica-alumina, silica, barium carbonate, barium sulfate, calcium carbonate, titanium oxide, zirconium oxide, zeolite, asbestos, and the like. Preferably, a metal such as platinum, rhodium, or palladium, or an alloy, a sulfide or a hydroxide of the metal is carried in the power support.

Examples of such a catalyst include platinum-carbon, platinum (II) sulfide-carbon, platinum-alumina, platinum-silica-alumina, platinum-silica, platinum-barium carbonate, platinum-barium sulfate, platinum-calcium carbonate, platinum-titanium oxide, platinum-zirconium oxide, platinum-zeolite, platinum-asbesto, platinum-rhodium alloy-carbon, platinum palladium alloy-carbon; rhodium-carbon, rhodium-alumina, rhodium-silica, rhodium-calcium carbonate; palladium-carbon, palladium (II) hydroxide-carbon, palladium (II) sulfide-carbon, palladium-alumina, palladium-silica-alumina, palladium-silica, palladium-barium carbonate, palladium-barium sulfate, palladium-calcium carbonate, palladium-titanium oxide, palladium-zirconium oxide, palladium-zeolite, palladium-asbesto; ruthenium-carbon, ruthenium-alumina, ruthenium-silica, ruthenium-calcium carbonate; iridium-carbon, iridium-alumina, iridium-silica, iridium-calcium carbonate, and the like. Preferably, palladium-carbon, rhodium-carbon, platinum-carbon, palladium-alumina, platinum-alumina, palladium-calcium carbonate, platinum-calcium carbonate, palladium-barium sulfate, platinum-barium sulfate, or the like can be used.

These transition metal catalysts may be used individually or in a combination of at least two catalysts. The amount of the transition metal catalyst used is a molar ratio of 1 or less, preferably 0.2 or less, and more preferably 0.1 or less, to the di- or tri-halohydroxyacetone derivative used.

Examples of the solvent to be used for the reaction include water; alcohol solvents such as methanol, ethanol, isopropanol, n-butanol, and the like; hydrocarbon solvents such as hexane, heptane, benzene, toluene, and the like; ether solvents such as diethyl ether, tetrahydrofuran, 1,4-dioxane, tert-butyl methyl ether, dimethoxyethane, and the like; ester solvents such as ethyl acetate, n-propyl acetate, tert-butyl acetate, and the like; ketone solvents such as acetone, methyl ethyl ketone, and the like; halogenated solvents such as methylene chloride, chloroform, 1,1,1-trichloroethane, and the like; nitrogen-containing solvents such as dimethylformamide, acetamide, formamide, acetonitrile, propionitrile, and the like; aprotic solvents such as dimethylsulfoxide, N-methylpyrrolidone, hexamethylphosphoric triamide, and the like. These reaction solvents may be used individually or in a combination of at least two solvents. Of these solvents, a protic solvent such as dimethylformamide, acetamide, formamide, water, or an alcohol solvent such as methanol, ethanol, or the like is preferably used, and water or an alcohol solvent such as methanol, ethanol, or the like is more preferably used.

The hydrogen pressure in the reaction is preferably 50 atm or less, and more preferably 10 atm or less. The lower limit of the hydrogen pressure is 1 atm or more.

The reaction temperature of the reaction is preferably - 20°C to 100°C, and more preferably 10°C to 50°C. The reaction time of the reaction is preferably 1 hour to 72 hours, and more preferably 1 hour to 12 hours.

As a post-treatment of the reaction, for example, a general post-treatment may be performed for obtaining the product from the reaction solution. For example, water is added to the reaction solution containing 1-chloro-3-hydroxyacetone, and extraction is performed with a general extraction solvent such as ethyl acetate, diethyl ether, methylene chloride, toluene, hexane, or the like. Then, the reaction solvent and the extraction solvent are distilled off by a heating operation under reduced pressure, or the like to obtain the target compound. When the transition metal catalyst insoluble in the reaction solvent is used, the transition metal catalyst is removed by an operation such as filtration under reduced pressure, pressure filtration, centrifugation, or the like, and then the reaction solvent is distilled off by an operation such as heating under reduced pressure or the like to obtain the target compound. The solution containing the product may be used in a next step.

Next, the method for isolating and purifying 2,5-dichloromethyl-2,5-dihydroxy-1,4-dioxane represented by formula (11) of the present invention will be described.

After the monohalohydroxyacetone derivative is extracted from the filtrate solution containing the monohalohydroxyacetone derivative with a general extraction solvent such as ethyl acetate, tert-butyl methyl ether, diethyl ether, methylene chloride or the like, 1-chloro-3-hydroxyacetone is dimerized to 2,5-dichloromethyl-2,5-dihydroxy-1,4-dioxane represented by formula (11) by a concentration or cooling operation or adding an auxiliary solvent such as hexane, toluene or the like, and then 2,5-dichloromethyl-2,5-dihydroxy-1,4-dioxane is isolated and purified as a crystal compound.

The compound (11) is liable to contain various impurities produced by decomposition and side reactions in the production process, such as acetol, 1,1-dichloro-3-hydroxyacetone, 2,5-di(dichloromethyl)-2,5-dihydroxy-1,4-dioxane, 1,3-dichloroacetone, 1,3-dihydroxyacetone, and the like. Particularly, the compound (11) tends to contain byproducts such as acetol, 1,1-dichloro-3-hydroxyacetone, 2,5-di(dichloromethyl)-2,5-dihydroxy-1,4-dioxane, and the like by a hydrogenation reaction, and thus these impurities must be removed for obtaining the high-quality target compound. It is generally difficult to remove impurities (analogues) having similar structures, and thus an excellent purification and isolation method is necessary for removing the impurities to obtain the high-quality target compound.

As a result of intensive research, the inventors developed a method capable of removing the impurities by crystallizing the compound (11) containing the impurities from an organic solvent to efficiently obtain the high-purity target material.

As the organic solvent used in this process, an organic solvent having a relatively low boiling point is preferably used in consideration of drying of the solvent of the wet crystals and recovery and recycle (distillation recovery) of the solvent. An organic solvent having a boiling point of about 100°C or less at 1 atom or less is generally used.

The organic solvent is not particularly limited. Examples of the organic solvent include aromatic hydrocarbon solvents such as benzene, toluene, o-xylene, m-xylene, p-xylene, ethylbenzene, cumene, n-butylbenzene, 1,3,5-mesitylene, and the like; ester solvents such as ethyl formate, methyl acetate, ethyl acetate, n-propyl acetate, n-butyl acetate, tert-butyl acetate, methyl propionate, ethyl propionate, γ-butyrolactone, and the like; ether solvents such as tert-butyl methyl ether, diethyl ether, diisopropyl ether, di-n-butyl ether, dimethoxyethane, diethylene glycol dimethyl ether, tetrahydrofuran, 1,4-dioxane, anisole, and the like; ketone solvents such as acetone, methyl ethyl ketone, diethyl ketone, cyclopentanone, cyclohexanone, and the like; nitrile solvents such as acetonitrile, propionitrile, and the like; halogenated solvents such as methylene chloride, chloroform, carbon tetrachloride, 1,2-dichloroethane, 1,1,1-trichloroethane, chlorobenzene, and the like; alcohol solvents such as methanol, ethanol, n-propanol, isopropanol, n-butanol, and the like.

Preferred examples include aromatic hydrocarbon solvents such as benzene, toluene, o-xylene, m-xylene, p-xylene, ethylbenzene, cumene, n-butylbenzene, 1,3,5-mesitylene, and the like; ester solvents such as ethyl formate, methyl acetate, ethyl acetate, n-propyl acetate, n-butyl acetate, tert-butyl acetate, methyl propionate, ethyl propionate, γ-butyrolactone, and the like; ether solvents such as tert-butyl methyl ether, diethyl ether, diisopropyl ether, di-n-butyl ether, dimethoxyethane, diethylene glycol dimethyl ether, tetrahydrofuran, and the like. From the comprehensive viewpoint of the solvent cost and handleability, toluene, ethyl acetate, tert-butyl methyl ether, and the like are more preferred. The organic solvents may be used individually or in a combination of at least two solvents.

By using the organic solvent, the effect of highly purifying the compound (11), i.e., the effect of effectively removing impurities, particularly analogues of the compound (11), can be achieved. The amount of the organic solvent used is preferably set to maintain the fluidity of the target material at the end of the operation of crystallizing the compound (11). For example, the amount of the organic solvent is preferably 30 times or less, more preferably about 1 to 30 times, the weight of the compound (11).

In the present invention, the compound (11) can be crystallized by a crystallization method such as cooling crystallization, concentration crystallization, or the like, or a combination of these crystallization methods. As the concentration crystallization method, a crystallization method comprising substituting a solution containing a solvent other than the organic solvent for a solution containing the organic solvent may be used. Also, in crystallization, seed crystals may be added.

In the present invention, in the crystallization, an auxiliary solvent besides the organic solvent may be further used for improving at least one of the yield, treatment concentration and liquidity of the compound (11), and the physical properties of the resultant crystals. According to need, the auxiliary solvent may be added to the organic solvent, or the compound (11) may be previously dissolved, by heating, in a mixed solution containing the auxiliary solvent and the organic solvent, and then crystallized by cooling.

The auxiliary solvent is not particularly limited. Examples of the auxiliary solvent include aliphatic hydrocarbon solvents such as pentane, petroleum ether, neopentane, hexane, cyclohexane, methyl cyclohexane, heptane, cycloheptane, octane, isooctane, nonane, decane, and the like. These solvents may be used individually or in a combination of at least two solvents. Particularly, from the comprehensive viewpoint of solvent drying of the wet material, recovery and recycle (distillation recovery) of the solvent, solvent cost, and handleability, hexane, toluene, or the like is preferred.

The appropriate amount of the auxiliary solvent used can be set by a simple experiment. From the viewpoint of yield and the fluidity of crystal slurry, the ratio by volume (organic solvent/auxiliary solvent) of the organic solvent to the auxiliary solvent is preferably 20 or less, and more preferably 10 or less, at the end of the operation of crystallizing the compound (11).

In the present invention, the purification and isolation method can be carried out near room temperature. According to need, heating or cooling may be performed at, for example, about 60°C or less, usually -30°C to 50°C.

After solid-liquid separation of the compound (11) obtained as described above, the resultant cake can be further washed and dried according to need. The solid-liquid separating method is not particularly limited, and for example, pressure filtration, filtration under reduced pressure, centrifugation, or the like may be used. As the drying method, for example, drying at about 60°C or less under reduced pressure (vacuum drying) is preferred for avoiding thermal decomposition and melting.

Next, the method for producing optically active halopropanediol derivatives of the present invention will be described.

The carbonyl group of a derivative represented by any one of formulae (3) to (6) is stereoselectively reduced in the presence of an enzyme source having the ability (activity) to stereoselectively reduce the carbonyl group of the halohydroxyacetone derivative represented by any one of formulae (3) to (6) to produce an optically active halopropanediol derivative represented by formula (7) or (8).

In the enzyme reducing step of the present invention, the enzyme source having the activity to stereoselectively reduce the carbonyl group of the halohydroxyacetone derivative is an enzyme source derived from at least one microorganisms belonging to a genus selected from the group consisting of Ambrosiozyma, Brettanomyces, Candida, Debaryomyces, Dipodascus, Geotrichum, Galactomyces, Issatchenkia, Kluyveromyces, Lodderomyces, Metschnikowia, Ogataea, Pichia, Rhodosporidium, Rhodotorula, Saccharomycopsis, Stephanoascus, Torulaspora, Trigonopsis, Trichosporon, Yamadazyma, Yarrowia, Cellulomonas, Enterobacter, Jensenia, Klebsiella, Microbacterium, Morganella, Nocardia, Rhodococcus, Serratia, Absidia, Acrotheca, Acremonium, Aegerita, Aspergillus, Auxarthron, Byssochlamys, Chaetomidium, Cladosporium, Corynascus, Corynespora, Cryptophiale, Coriolus, Dendryphiella, Fistulina, Fusarium, Gibberella, Macrophoma, Mortierella, Mucor, Neocosmospora, Paecilomyces, Panus, Penicillium, Pleurotus, Plectosphaerella, Rhizopus, Sclerotinia, Sclerotium, Scopulariopsis, Schizophyllum, Sphaerodes, Tyromyces, Verticillium, Wardomyces and Streptomyces.

The enzyme source is preferably derived from at least one microorganisms selected from the group consisting of Ambrosiozyma philentoma IFO1847, Brettanomyces anomalus IFO0627, Candida etchellsii IFO1229, Candida gropengiesseri IFO0659 Candida magnoliae IFO0705 Candida maris IFO10003 Candida tenuis IFO0716, Debaryomyces hansenii IFO0063, Debaryomyces hansenii var. hansenii IFO0032, Debaryomyces robertsiae IFO1277, Dipodascus ovetensis IFO1201, Dipodascus tetrasperma IFO10810, Geotrichum fermentans IFO1199, Galactomyces reessii IFO10823, Issatchenkia terricola IFO0933, Kluyveromyces polysporus IFO0996, Kluyveromyces thermotolerans IFO0662, Lodderomyces elongisporus IFO1676, Metschnikowia bicuspidata var. bicuspidata IFO1408, Ogataea minuta var. minuta IFO0975, Pichia bovis IFO0872, Pichia anomala IFO0120, Pichia silvicola IFO0807, Rhodosporidium toruloides IFO0388, Rhodotorula aurantiaca IFO0754, Rhodotorula araucariae IFO10053, Rhodotorula lactosa IFO1423, Saccharomycopsis selenospora IFO1850, Saccharomycopsis vini IFO1748, Stephanoascus ciferrii IFO1854, Torulaspora delbrueckii IFO0381, Trigonopsis variabilis IFO0671, Trichosporon asteroides IFO0173, Yamadazyma stipitis IFO10063, Yamadazyma haplophila IFO0947, Yarrowia lipolytica IFO0746, Cellulomonas sp. JCM2471, Cellulomonas uda IFO3747, Cellulomonas fimi IFO15513, Enterobacter aerogenes IFO13534, Jensenia canicruria IFO13914, Klebsiella planticola IFO3317, Klebsiella pneumoniae subsp. pneumoniae IFO3319, Microbacterium arborescens IFO3750, Microbacterium esteraromaticum IFO3752, Morganella morganii subsp. morganii IFO3848, Nocardia globerula IFO13510, Rhodococcus erythropolis IFO12320, Rhodococcus equi IFO3730, Rhodococcus rhodochrous IFO3338, Serratia marcescens IFO12648, Absidia coerulea IFO4011, Acrotheca cerophila IFO6881, Acremonium butyri IFO7826, Aegerita candida IFO6988, Aspergillus versicolor IFO30338, Aspergillus sydowii IFO4284, Auxarthron thaxteri IFO8451, Byssochlamys fulva IFO6307, Chaetomidium fimeti IFO30419, Cladosporium resinae IFO8588, Corynascus sepedonium IFO30067, Corynespora cassiicola IFO30049, Cryptophiale guadalcanalense IFO30029, Coriolus consors IFO9078, Dendryphiella salina IFO8281, Fistulina hepatica IFO30300, Fusarium oxysporum IFO5942, Fusarium anguioides IFO4467, Gibberella fujikuroi IFO6603, Macrophoma commelinae IFO9569, Mortierella vinacea IFO6738, Mucor tuberculisporus IFO9256, Mucor inaequisporus IFO8624, Neocosmospora vasinfecta IFO30064, Paecilomyces lilacinus IFO31914, Panus lacomtei IFO31653, Penicillium janthinellum IFO4651, Pleurotus ostreatus IFO6515, Plectosphaerella cucumerina IFO30005, Rhizopus niveus IFO4759, Rhizopus oryzae IFO4705, Rhizopus stolonifer var. stolonifer IFO4781, Sclerotinia sclerotiorum IFO4876, Sclerotium delphinii IFO7337, Scopulariopsis brevicaulis IFO4843, Schizophyllum commune IFO6503, Sphaerodes fimicola IFO8354, Tyromyces palustris IFO30339, Verticillium niveostratosum IFO5435, Wardomyces anomalus IFO8284, Streptomyces cacaoi subsp. asoensis IFO13813, Streptomyces celluloflavus IFO13780, Streptomyces coelescens IFO13378, Streptomyces diastatochromogenes IFO13389, Streptomyces lividans ATCC35287, Streptomyces hydrogenans IFO13475, Streptomyces achromogenes subsp. rubradiris IFO14000, and Streptomyces salmonis IFO15865.

Also, the enzyme source may be an enzyme source classified in glycerol dehydrogenase (EC 1.1.1.6). Such an enzyme source is preferably glycerol dehydrogenase derived from at least one microorganisms belonging to a genus selected from the group consisting of Serratia and Cellulomonas.

Of these enzyme sources, the enzyme source having the ability to (R)-selectively reduce the carbonyl groups of halohydroxyacetone derivatives is derived from at least one microorganisms belonging to a genus selected from the group consisting of Brettanomyces, Candida, Debaryomyces, Dipodascus, Geotrichum, Galactomyces, Issatchenkia, Kluyveromyces, Lodderomyces, Ogataea, Pichia, Rhodosporidium, Rhodotorula, Saccharomycopsis, Stephanoascus, Torulaspora, Trigonopsis, Trichosporon, Yamadazyma, Yarrowia, Enterobacter, Klebsiella, Microbacterium, Absidia, Acrotheca, Acremonium, Aegerita, Aspergillus, Chaetomidium, Cladosporium, Corynespora, Cryptophiale, Fusarium, Gibberella, Macrophoma, Mucor, Neocosmospora, Paecilomyces, Penicillium, Pleurotus, Plectosphaerella, Rhizopus, Sclerotinia, Sclerotium, Sphaerodes, Tyromyces, Verticillium, and Streptomyces.

The enzyme source is preferably derived from microorganisms belonging to a genusof Candida, and specifically the enzyme source is derived from a microorganism selected from the group consisting of Candida magnoliae and Candida maris.

The enzyme source having the ability to (S)-selectively reduce the carbonyl groups of halohydroxyacetone derivatives is an enzyme source derived from at least one microorganisms belonging to a genus selected from the group consisting of Ambrosiozyma, Candida, Kluyveromyces, Metschnikowia, Yamadazyma, Cellulomonas, Jensenia, Microbacterium, Morganella, Nocardia, Rhodococcus, Serratia, Aspergillus, Auxarthron, Byssochlamys, Corynascus, Coriolus, Dendryphiella, Fistulina, Mortierella, Panus, Scopulariopsis, Schizophyllum, Wardomyces and Streptomyces.

The enzyme source is preferably derived from at least one microorganisms belonging to a genus selected from the group consisting of Candida, Serratia, Cellulomonas, and specifically derived from microorganisms of at least one genus selected from Candida magnoliae and Serratia marcescens.

The enzyme source having the ability to (S)-selectively reduce the carbonyl groups of halohydroxyacetone derivatives may be glycerol dehydrogenase, and more specifically glycerol dehydrogenase derived from microorganisms of at least one genus selected from the group consisting of Cellulomonas and Serratia.

Microorganisms having the ability to produce a microorganism-derived reductase may be of a wild strain or a variant. Microorganisms induced by a genetic method such as cell fusion, genetic manipulation, or the like also can be used. The enzyme-producing microorganisms produced by genetic manipulation can be produced by a method comprising the steps of isolating and/or purifying the enzyme and determining a part or the whole of the amino acid sequence of the enzyme, producing a DNA sequence coding for the enzyme based on the amino acid sequence, introducing the DNA into other microorganisms to produce recombinant microorganisms, and culturing the recombinant microorganisms to produce the enzyme (WO98/35025).

A culture medium for the microorganisms used as the enzyme source is not particularly limited as long as the microorganisms can proliferate. For example, a usual liquid medium containing a carbon source, a nitrogen source, inorganic salts, and another nutrient source can be used.

Examples of the carbon source include glucides such as glucose, sucrose, and the like; alcohols such as ethanol, glycerol, and the like; fatty acids such as oleic acid, stearic acid, and the like, and esters thereof; oils such as rapeseed oil, soybean oil, and the like. Examples of the nitrogen source include ammonium sulfate, sodium nitrate, peptone, casamino acid, corn steep liquor, wheat bran, yeast extract, and the like. Examples of the inorganic salts include magnesium sulfate, sodium chloride, calcium carbonate, potassium hydrogen phosphate, potassium dihydrogen phosphate, and the like. Examples of the other nutrient source include malt extract, meat extract, and the like.

Culturing can be aerobically performed usually for a culture time of about 1 to 5 days, preferably 1 to 3 days, at a culture temperature of 10°C to 50°C, preferably 20°C to 40°C, with a medium pH of 3 to 9, preferably 5 to 8.

In the reduction step of the present invention, a halohydroxyacetone derivative used as a substrate, a coenzyme NAD(P)H, and a cultured product or treated product of the microorganisms are added to an appropriate solvent, and then the resultant mixture is stirred under pH control to effect a reaction.

Although the reaction conditions depend upon the enzyme used, the microorganisms or a treated product thereof used, and the substrate concentration, the substrate concentration is usually about 0.1% by weight to 100% by weight, preferably 1% by weight to 60% by weight, and the content of the coenzyme NAD(P)H is 0.0001 to 100 mol%, preferably 0.0001 to 0.1 mol% based on the substrate. The reaction temperature is 10°C to 60°C, preferably 20°C to 50°C, the reaction pH is 4 to 9, preferably 5 to 8, and the reaction time is 1 hour to 120 hours, preferably 1 hour to 72 hours. The substrate can be simultaneously or successively added. The reaction can be effected in a batch system or a continuous system.

The "cultured product of the microorganisms" means a culture broth containing bacterial cells or cultured bacterial cells, and the "treated product" means, for example, a crude extract, lyophilized microorganisms, acetone-dried microorganisms, ground microorganisms, or the like. Furthermore, the enzyme itself or the bacterial cells can be immobilized as they are by a known means. Immobilization can be performed by a method known by persons skilled in the art (for example, a crosslinking method, a physical adsorption method, an entrapment method, or the like) .

In the reduction step of the present invention, a combination with a generally used coenzyme NAD(P)H regenerating system can significantly decrease the amount of the expensive coenzyme used. A typical example of the NAD(P)H regenerating system is a system using glucose dehydrogenase and glucose.

The above-described reaction may be effected by using a cultured product or a treated product of a transformant which is produced by introducing, into same host microorganisms, a reductase gene and a gene of an enzyme (for example, glucose dehydrogenase) having the ability to regenerate a coenzyme on which the enzyme depends. In this case, another enzyme source necessary for regenerating the coenzyme need not be prepared, and thus an optically active halopropanediol derivative can be produced at low cost.

As the transformant, a transformant transformed with a plasmid having a DNA coding for the reductase and a DNA coding for an enzyme having the ability to regenerate the coenzyme on which the enzyme depends can be used.

Preferred examples of the transformant include a transformant produced by using glucose dehydrogenase as an enzyme having the ability to regenerating the coenzyme; a transformant produced by the glucose dehydrogenase derived from Bacillus megaterium; a transformant produced by using, as the plasmid, pNTS1G (having a DNA coding for the reductase derived from Candida magnoliae IFO0705 and a DNA coding for the glucose dehydrogenase derived from Bacillus megaterium), pNTCRG (having a DNA coding for the reductase derived from Candida magnoliae IFO0705 and a DNA coding for the glucose dehydrogenase derived from Bacillus megaterium), pNTFPG (having a DNA coding for the reductase derived from Candida maris IFO10003 and a DNA coding for the glucose dehydrogenase derived from Bacillus megaterium), or pNTSGG1 (having a DNA coding for the reductase derived from Serratia marcescens IFO12468 and a DNA coding for the glucose dehydrogenase derived from Bacillus megaterium); a transformant produced by using Escherichia coli bacteria as the host microorganisms.

More preferable examples of the enzyme source include Escherichia coli HB101 (pNTS1G) Accession No. FERM BP-5835, Escherichia coli HB101 (pNTCRG) Accession No. FERM BP-6898, Escherichia coli HB101 (pNTFPG) Accession No. FERM BP-7117, and Escherichia coli HB101 (pNTSGG1) Accession No. FERM P-18449.

At National Institute of Advanced Industrial Science and Technology, International Patent Organism Depositary, Tsukuba Central 6, 1-1-1, Higashi, Tsukuba-shi, Ibaraki, Japan, Escherichia coli HB101 (pNTS1G) was deposited as Accession No. FERM BP-5835 on February 24, 1997, Escherichia coli HB101 (pNTCRG) was deposited as Accession No. FERM BP-6898 on September 28, 1999, Escherichia coli HB101 (pNTFPG) was deposited as Accession No. FERM BP-7117 on April 11, 2000, and Escherichia coli HB101 (pNTSGG1) was deposited as Accession No. FERM P-18449 on August 6, 2001.

Of these enzyme sources, the enzyme source having the ability to (R)-selectively reduce the carbonyl groups of halohydroxyacetone derivative is Escherichia coli HB101 (pNTS1G) Accession No. FERM BP-5835 or Escherichia coli HB101 (pNTFPG) Accession No. FERM BP-7117, and the enzyme source having the ability to (S)-selectively reduce the carbonyl groups is Escherichia coli HB101 (pNTCRG) Accession No. FERM BP-6898 or Escherichia coli HB101 (pNTSGG1) Accession No. FERM P-18449.

In the present invention, when the reduction step is performed in a combination with the coenzyme regenerating system or by using a cultured product of the transformant or a treated product thereof as the enzyme source, inexpensive oxidized NAD(P) can be added as the coenzyme for the reaction.

The optically active halopropanediol derivatives produced by the reduction reaction can be purified by a usual method. For example, optically active 3-chloro-1,2-propanediol can be purified by a method comprising removing suspended materials such as bacterial cells or the like by a treatment such as centrifugation, filtration or the like according to need when microorganisms are used, performing extraction with an organic solvent such as ethyl acetate, toluene or the like, removing the organic solvent under reduced pressure, and then purifying the residue by distillation under reduced pressure or chromatography, or the like.

Best Mode for Carrying Out the Invention

Although the present invention will be described in further detail below with reference to examples, the present invention is not limited to these examples.

(Example 1)

A liquid medium (pH 7.0) containing 4% of glucose, 0.3% of yeast extract, 1.3% of $KH_2PO_4$, 0.7% of $(NH_4)_2HPO_4$, 0.01% of NaCl, 0.08% of $MgSO_4 \cdot 7H_2O$, 0.006% of $ZnSO_4 \cdot 7H_2O$, 0.009% of $FeSO_4 \cdot 7H_2O$, 0.0005% of $CuSO_4 \cdot 5H_2O$, and 0.001% of $MnSO_4 \cdot 4\text{-}5H_2O$ was prepared, and 5 ml of the liquid medium was injected into each of large test tubes and then sterilized with steam at 120°C for 20 minutes. Then, one platinum loop of the microorganisms of each of the genera shown in Table 1 and 2 was inoculated into the liquid medium, and cultured with shaking at 30°C for 2 to 3 days. Cells were collected from each of the culture broths by centrifugation, washed with water and then suspended in 1 ml of a 0.1M phosphate buffer (pH 5.5). Then, 0.5 ml of the cell suspension was mixed with 0.5 ml of a 0.1M $KH_2PO_4$ aqueous solution containing 5 mg of 1-chloro-3-hydroxyacetone and 20 mg of glucose, and the resultant mixture was placed in a test tube with a stopper and shaken at 30°C for 24 hours. After completion of reaction, a saturated amount of ammonium sulfate was added to the reaction solution, and extraction was performed with an equivalent volume of ethyl acetate. The amounts of the substrate and product in the extract were analyzed by gas chromatography (GC) to determine a conversion rate (%) and optical purity (%ee). For a sample not sufficiently separated, the optical purity was determined by high-performance liquid chromatography (HPLC) after the primary hydroxyl group of the product was tosylated with tosyl chloride.

The analysis conditions and the methods for calculating the conversion rate and optical purity were as follows.

GC analysis conditions = column: CHIRALDEX G-TA 20m×0.25mm I.D. (produced by ASTEC), carrier gas: He, detection: FID, column temperature: 85°C, detection time: 1-chloro-3-hydroxyacetone 6.2 minutes, 3-chloro-1,2-propanediol S-isomer: 11.2 minutes, R-isomer: 12.0 minutes.

HPLC analysis conditions = column: Chiralpak AD (produced by Daicel Chemical Industries, Ltd.), eluant: hexane/isopropanol = 9/1, flow rate: 0.8 ml/min, detection: 235nm, column temperature: room temperature, detection time: S-isomer 29 minutes, R-isomer 35 minutes.

Conversion rate (%) = amount of product/(amount of substrate + amount of product) x 100

Optical purity (%ee) = (A - B)/(A + B) x 100 (A and B respectively represent the amounts of corresponding enantiomers, and A > B).

Table 1

| Microorganisms | Conversion rate (%) | Optical purity (%e.e.) | Configuration |
|---|---|---|---|
| Ambrosiozyma philentoma IFO 1847 | 6.6 | 76.6 | S |
| Brettanomyces anomalus IFO 0627 | 61.6 | 82.9 | R |
| Candida etchellsii IFO 1229 | 45.8 | 90.0 | R |
| Candida gropengiesseri IFO 0659 | 99.7 | 78.8 | R |
| Candida tenuis IFO 0716 | 12.1 | 83.1 | S |
| Debaryomyces hansenii IFO 0063 | 39.4 | 92.0 | R |
| Debaryomyces hansenii var. hansenii IFO 0032 | 59.6 | 89.8 | R |

Table 1 (continued)

| Microorganisms | Conversion rate (%) | Optical purity (%e.e.) | Configuration |
|---|---|---|---|
| Debaryomyces robertsiae IFO 1277 | 47.2 | 91.6 | R |
| Dipodascus ovetensis IFO 1201 | 26.3 | 91.1 | R |
| Dipodascus tetrasperma IFO 10810 | 31.0 | 4.8 | R |
| Geotrichum fermentans IFO 1199 | 9.7 | 89.6 | R |
| Galactomyces reessii IFO 10823 | 38.3 | 87.1 | R |
| Issatchenkia terricola IFO 0933 | 51.7 | 67.9 | R |
| Kluyveromyces polysporus IFO 0996 | 31.1 | 93.7 | S |
| Kluyveromyces thermotolerans IFO 0662 | 16.0 | 67.5 | R |
| Lodderomyces elongisporus IFO 1676 | 11.2 | 59.8 | R |
| Metschnikowia bicuspidata var. bicuspidata IFO 1408 | 22.3 | 67.3 | S |
| Nakazawaea holstii IFO 0980 | 6.2 | 0.0 | - |
| Ogataea minuta var. minuta IFO 0975 | 7.9 | 3.6 | R |

Table 2

| Microorganisms | Conversion rate (%) | Optical purity (%e.e.) | Configuration |
|---|---|---|---|
| Pichia bovis IFO 0872 | 8.9 | 57.5 | R |
| Pichia anomala IFO 0120 | 7.4 | 20.7 | R |
| Pichia silvicola IFO 0807 | 13.9 | 29.6 | R |
| Rhodosporidium toruloides IFO 0388 | 31.3 | 32.1 | R |
| Rhodotorula aurantiaca IFO 0754 | 7.0 | 57.3 | R |
| Rhodotorula araucariae IFO 10053 | 93.2 | 49.6 | R |
| Rhodotorula lactosa IFO 1423 | 21.6 | 20.0 | R |
| Saccharomycopsis selenospora IFO 1850 | 9.3 | 69.3 | R |
| Saccharomycopsis vini IFO 1748 | 27.3 | 78.0 | R |
| Stephanoascus ciferrii IFO 1854 | 6.1 | 82.1 | R |
| Torulaspora delbrueckii IFO 0381 | 20.8 | 91.0 | R |
| Trigonopsis variabilis IFO 0671 | 6.5 | 19.3 | R |
| Trichosporon asteroides IFO 0173 | 14.5 | 6.0 | R |
| Yamadazyma stipitis IFO 10063 | 77.8 | 81.2 | S |
| Yamadazyma haplophila IFO 0947 | 9.5 | 38.0 | R |
| Yarrowia lipolytica IFO 0746 | 43.8 | 76.8 | R |

(Example 2)

For the microorganisms shown in Table 3, the same operations as those in Example 1 were carried out except that a liquid medium (pH 7.0) containing 1.5% of glycerin, 0.5% of yeast extract, 1.3% of $KH_2PO_4$, 0.7% of $(NH_4)_2HPO_4$, 0.01% of NaCl, 0.08% of $MgSO_4 \cdot 7H_2O$, 0.006% of $ZnSO_4 \cdot 7H_2O$, 0.009% of $FeSO_4 \cdot 7H_2O$, 0.0005% of $CuSO_4 \cdot 5H_2O$,

and 0.001% of $MnSO_4.4\text{-}5H_2O$ was used, to measure conversion rates and optical purities. The results are shown in Table 3.

Table 3

| Microorganisms | Conversion rate (%) | Optical purity (%e.e.) | Configuration |
|---|---|---|---|
| Cellulomonas sp. JCM 2471 | 87.8 | 88.3 | S |
| Cellulomonas uda IFO 3747 | 5.1 | 47.3 | S |
| Cellulomonas fimi IFO 15513 | 68.1 | 83.5 | S |
| Enterobacter aerogenes IFO 13534 | 4.6 | 50.5 | R |
| Jensenia canicruria IFO 13914 | 50.2 | 44.1 | S |
| Klebsiella planticola IFO 3317 | 4.3 | 39.0 | R |
| Klebsiella pneumoniae subsp. pneumoniae IFO 3319 | 3.2 | 28.2 | R |
| Microbacterium arborescens IFO 3750 | 7.9 | 17.5 | R |
| Microbacterium esteraromaticum IFO 3752 | 21.2 | 46.7 | S |
| Morganella morganii subsp. morganii IFO 3848 | 19.8 | 91.3 | S |
| Nocardia globerula IFO 13510 | 4.1 | 41.9 | S |
| Rhodococcus erythropolis IFO 12320 | 71.9 | 72.0 | S |
| Rhodococcus equi IFO 3730 | 8.5 | 89.0 | S |
| Rhodococcus rhodochrous IFO 3338 | 2.8 | 77.9 | S |

(Example 3)

For the microorganisms shown in Tables 4 and 5, the same operations as in Example 1 were performed except that a liquid medium (pH 7.0) containing 1% of meat extract, 1% of peptone, 1% of glucose, 0.5% of yeast extract, 0.1% of NaCl, and 0.05% of $MgSO_4.7H_2O$ was used, to measure conversion rates and optical purities. The results are shown in Tables 4 and 5.

Table 4

| Microorganisms | Conversion rate (%) | Optical purity (%e.e.) | Configuration |
|---|---|---|---|
| Absidia coerulea IFO 4011 | 76.7 | 95.4 | R |
| Acrotheca cerophila IFO 6881 | 73.2 | 96.6 | R |
| Acremonium butyri IFO 7826 | 71.5 | 91.3 | R |
| Aegerita candida IFO 6988 | 97.9 | 92.0 | R |
| Aspergillus versicolor IFO 30338 | 7.2 | 35.0 | R |
| Aspergillus sydowii IFO 4284 | 4.8 | 34.0 | S |
| Auxarthron thaxteri IFO 8451 | 8.5 | 15.0 | S |
| Byssochlamys fulva IFO 6307 | 21.6 | 88.5 | S |
| Chaetomidium fimeti IFO 30419 | 99.9 | 78.3 | R |
| Cladosporium resinae IFO 8588 | 4.7 | 5.1 | R |
| Corynascus sepedonium IFO 30067 | 79.8 | 83.7 | S |
| Corynespora cassiicola IFO 30049 | 27.5 | 75.2 | R |
| Cryptophiale guadalcanalense IFO 30029 | 15.8 | 40.8 | R |

Table 4 (continued)

| Microorganisms | Conversion rate (%) | Optical purity (%e.e.) | Configuration |
|---|---|---|---|
| Coriolus consors IFO 9078 | 27.8 | 28.4 | S |
| Dendryphiella salina IFO 8281 | 99.9 | 59.3 | S |
| Fistulina hepatica IFO 30300 | 1.2 | 64.7 | S |
| Fusarium oxysporum IFO 5942 | 93.9 | 59.5 | R |
| Fusarium anguioides IFO 4467 | 6.7 | 76.2 | R |
| Gibberella fujikuroi IFO 6603 | 16.1 | 52.8 | R |
| Macrophoma commelinae IFO 9569 | 5.8 | 77.4 | R |
| Mortierella vinacea IFO 6738 | 30.5 | 25.7 | S |

Table 5

| Microorganisms | Conversion rate (%) | Optical purity (%e.e.) | Configuration |
|---|---|---|---|
| Mucor tuberculisporus IFO 9256 | 65.8 | 96.1 | R |
| Mucor inaequisporus IFO 8624 | 78.8 | 96.8 | R |
| Neocosmospora vasinfecta IFO 30064 | 56.6 | 63.4 | R |
| Paecilomyces lilacinus IFO 31914 | 52.5 | 94.2 | R |
| Panus lacomtei IFO 31653 | 1.7 | 61.3 | S |
| Penicillium janthinellum IFO 4651 | 10.5 | 45.3 | R |
| Pleurotus ostreatus IFO 6515 | 6.3 | 82.2 | R |
| Plectosphaerella cucumerina IFO 30005 | 35.7 | 82.0 | R |
| Rhizopus niveus IFO 4759 | 29.5 | 45.8 | R |
| Rhizopus oryzae IFO 4705 | 37.0 | 16.1 | R |
| Rhizopus stolonifer var. stolonifer IFO 4781 | 34.0 | 3.1 | R |
| Sclerotinia sclerotiorum IFO 4876 | 8.6 | 8.0 | R |
| Sclerotium delphinii IFO 7337 | 5.0 | 10.6 | R |
| Scopulariopsis brevicaulis IFO 4843 | 24.9 | 66.6 | S |
| Schizophyllum commune IFO 6503 | 7.3 | 36.2 | S |
| Sphaerodes fimicola IFO 8354 | 25.4 | 66.1 | R |
| Tyromyces palustris IFO 30339 | 31.9 | 0.3 | R |
| Verticillium niveostratosum IFO 5435 | 26.2 | 94.0 | R |
| Wardomyces anomalus IFO 8284 | 98.8 | 47.2 | S |

(Example 4)

For the microorganisms shown in Table 6, the same operations as those in Example 1 were performed except that a liquid medium (pH 7.2) containing 3% of tryptotic soy broth and 1% of soluble starch was used, to measure conversion rates and optical purities. The results are shown in Table 6.

Table 6

| Microorganisms | Conversion rate (%) | Optical purity (%e.e.) | Configuration |
|---|---|---|---|
| Streptomyces cacaoi subsp.asoensis IFO 13813 | 11.4 | 68.2 | S |
| Streptomyces celluloflavus IFO 13780 | 4.3 | 68.5 | S |
| Streptomyces coelescens IFO 13378 | 19.9 | 61.2 | R |
| Streptomyces diastatochromogenes IFO 13389 | 5.0 | 75.3 | S |
| Streptomyces lividans ATCC 35287 | 32.1 | 54.0 | R |
| Streptomyces hydrogenans IFO 13475 | 5.9 | 73.4 | S |
| Streptomyces achromogenes subsp. rubradiris IFO 14000 | 4.0 | 76.3 | S |
| Streptomyces salmonis IFO 15865 | 5.1 | 73.8 | S |

(Example 5) Method for synthesizing 1,1-dichloro-3-hydroxyacetone

33.6 g of propargyl alcohol was added to 250 ml of a 20% sulfuric acid solution under ice cooling, and then 740 ml of a 13% sodium hypochlorite solution was added dropwisely to the resultant mixture over 2 hours, and further stirred for 2 hours. The reaction solution was controlled to pH 2 with 6N sodium hydroxide, and then extracted with ethyl acetate and concentrated under reduced pressure to obtain a light-yellow oily concentrate. The resultant concentrate was purified by a silica gel column to obtain the title compound as a slightly-yellow oily material.

$^1$H-NMR ($D_2O$, 400 MHz/ppm); 3.62 (2H, bs), 4.66 (2H, s), 5.88 (1H, s)

(Example 6) Method for synthesizing 2,5-di(dichloromethyl)-2,5-dihydroxy-1,4-dioxane

33.6 g of propargyl alcohol was added to 250 ml of a 20% sulfuric acid solution under ice cooling, and then 740 ml of a 13% sodium hypochlorite solution was added dropwisely to the resultant mixture over 2 hours, and further stirred for 2 hours. Then, the reaction solution was concentrated under reduced pressure to obtain a cloudy solution, which was then further stirred for 12 hours under ice cooling. As a result, the reaction solution was changed to a slurry state. The slurry was filtered to obtain the title compound as white crystals.

$^1$H-NMR ($CDCl_3$, 400 MHz/ppm); 3.20 (2H, bs), 3.70 (1H, d), 4.08 (1H, d), 5.78 (1H, s)

(Example 7) Method for synthesizing 1-chloro-3-hydroxyacetone

14.3 g of 2,5-di(dichloromethyl)-2,5-dihydroxy-1,4-dioxane obtained in Example 6 and 0.7g of 10% palladium-carbon were added to 100 ml of water, and the reactor was replaced with a hydrogen atmosphere (normal pressure). Then, the reaction solution was stirred at 40°C for 8 hours, and then filtered. The filtrate was controlled to pH 2 with 6N sodium hydroxide, extracted with ethyl acetate and then concentrated under reduced pressure to obtain a light yellow oily concentrate. The resultant concentrate was purified by a silica gel column to obtain the title compound as a slightly-yellow oily material.

(Example 8) Method for isolating and purifying 2,5-dichloromethyl-2,5-dihydroxy-1,4-dioxane

14.3 g of 2,5-di(dichloromethyl)-2,5-dihydroxy-1,4-dioxane obtained in Example 6 and 0.7g of 10% palladium-carbon were added to 100 ml of water, and the reactor was replaced with a hydrogen atmosphere (normal pressure). Then, the reaction solution was stirred at 30°C for 8 hours, and then filtered. The filtrate was controlled to pH 2 with 6N sodium hydroxide, extracted with ethyl acetate and then concentrated under reduced pressure to obtain a light yellow oily concentrate (1-chloro-3-hydroxyacetone content = 70%, 1,1-dichloroacetone content = 20%, acetol content = 7%: analyzed by the method described in Example 9 below). The resultant concentrate was dissolved in tert-butyl methyl ether, and hexane was then added to the resultant solution to cloud the reaction solution. The reaction solution was further stirred for 12 hours under ice cooling to change the reaction solution to a slurry state. The slurry was then filtered to obtain the tile compound as white crystals (content of 2,5-dichloromethyl-2,5-dihydroxy-1,4-dioxane = 99%).

$^1$H-NMR ($CDCl_3$+MeOD, 400 MHz/ppm); 3.50 (2H,bs), 3.65 (1H,d), 4.00 (2H,s), 4.08 (1H,d)

(Example 9) Method for synthesizing (S)-3-chloro-1,2-propanediol

A Serratia-derived glycerol dehydrogenase solution was obtained from a culture broth of Serratia marcescens IFO12648 strains by a purification operation comprising ammonium sulfate fractionation and ion-exchange chromatography. 1 ml of a 0.1M phosphate buffer (pH 6.5) containing 10 μl of the thus-obtained glycerol dehydrogenase solution, 1.5 mg of 1-chloro-3-hydroxyacetone, and 10 mg of NADH was stirred at 30°C for 2 hours. After the reaction was completed, the reaction solution was saturated with ammonium sulfate, and extracted with an equivalent volume of ethyl acetate. The amounts of the substrate and product in the extract were measured by gas chromatography (GC) analysis to determine the conversion rate (%). Also, the optical purity (%ee) of the product was measured by high-performance liquid chromatography (HPLC) analysis after the primary hydroxyl group of the product was tosylated with tosyl chloride. As a result, (S)-3-chloro-1,2-propanediol was produced with a conversion rate of 100% and an optical purity of 100%ee. The analysis conditions and the methods for calculating the conversion rate and the optical purity were as follows.

GC analysis conditions = column: PEG-20M 10% Chromsorb WAW DMCS 80/100 (produced by GL Sciences Inc.), $H_2$: 1.5 kg/cm$^2$, $N_2$: 0.5 kg/cm$^2$, Air: 1.0 kg/cm$^2$, detection: FID, column temperature: 150°C, elution time: 1-chloro-3-hydroxyacetone 2.4 minutes, 3-chloro-1,2-propanediol 5.7 minutes.

HPLC analysis conditions = column: Chiralpak AD (produced by Daicel Chemical Industries, Ltd.), eluant: hexane/isopropanol = 9/1, flow rate: 0.8 ml/min, detection: 235nm, column temperature: room temperature, elution time: S-isomer 29 minutes, R-isomer 35 minutes.

Conversion rate (%) = amount of product/(amount of substrate + amount of product) × 100

Optical purity (%ee) = (A - B)/(A + B) × 100 (A and B respectively represent the amounts of corresponding enantiomers, and A > B).

(Example 10) Method for synthesizing (S)-3-chloro-1,2-propanediol

1 ml of a 0.1M phosphate buffer (pH 6.5) containing 0.05 mg of Cellulomonas-derived glycerol dehydrogenase (produced by SIGMA), 1.5 mg of 1-chloro-3-hydroxyacetone, and 10 mg of NADH was stirred at 30°C for 2 hours. After the reaction was completed, the conversion rate and optical purity of the reaction product (S)-3-chloro-1,2-propanediol were analyzed by the same method as in Example 9. As a result, the conversion rate was 100%, and the optical purity was 99.7%ee.

(Example 11) Method for synthesizing (R)-3-chloro-1,2-propanediol

1 ml of 0.1M phosphate buffer (pH 6.5) containing 10 μl of a solution of Candida-derived reductase obtained by the method described in WO01/05996, 1.5 mg of 1-chloro-3-hydroxyacetone, and 10 mg of NADH was stirred at 30°C for 2 hours. After the reaction was completed, the conversion rate and optical purity of the reaction product (R)-3-chloro-1,2-propanediol were analyzed by the same method as in Example 9. As a result, the conversion rate was 100%, and the optical purity was 99.6%ee.

(Example 12) Method for synthesizing (S)-3-chloro-1,2-propanediol

Recombinant Escherichia coli HB101 (pNTSGG1) Accession No. FERM P-18449 were inoculated into 50 ml of sterilized 2×YT medium (16 g of tryptone, 10 g of yeast extract, 5 g of sodium chloride, and 1L of water, pH 7.0 before sterillization) in a 500 ml Sakaguchi flask, and cultured with shaking at 37°C for 18 hours. Then, 10 mg of 1-chloro-3-hydroxyacetone, 0.6 mg of $NAD^+$, and 25 mg of glucose were added to 1 ml of the resultant culture broth, and the mixture was then stirred at 30°C for 2 hours. After the reaction was completed, the conversion rate and optical purity of the reaction product (S)-3-chloro-1,2-propanediol were analyzed by the same method as in Example 9. As a result, the conversion rate was 97.1%, and the optical purity was 100%ee.

(Example 13) Method for synthesizing (S)-3-chloro1,2-propanediol

Recombinant Escherichia coli HB101 (pNTCRG) Accession No. FERM BP-6898 were inoculated into 50 ml of sterilized 2×YT medium (16 g of tryptone, 10 g of yeast extract, 5 g of sodium chloride, and 1L of water, pH 7.0 before sterillization) in a 500 ml Sakaguchi flask, and cultured with shaking at 37°C for 18 hours. Then, 10 mg of 1-chloro-3-hydroxyacetone, 70 µg of $NAD^+$, and 25 mg of glucose were added to 1 ml of the resultant culture broth, and the mixture was then stirred at 30°C for 2 hours. After the reaction was completed, the conversion rate and optical purity of the reaction product (S)-3-chloro-1,2-propanediol were analyzed by the same method as in Example 9. As a result, the conversion rate was 42.8%, and the optical purity was 45.8%ee.

(Example 14) Method for synthesizing (R)-3-chloro-1,2-propanediol

Recombinant Escherichia coli HB101 (pNTFPG) Accession No. FERM BP-7117 were inoculated into 50 ml of sterilized 2×YT medium (16 g of tryptone, 10 g of yeast extract, 5 g of sodium chloride, and 1L of water, pH 7.0 before sterillization) in a 500 ml Sakaguchi flask, and cultured with shaking at 37°C for 18 hours. Then, 10 mg of 1-chloro-3-hydroxyacetone, 70 µg of $NAD^+$, and 25 mg of glucose were added to 1 ml of the resultant culture broth, and the mixture was then stirred at 30°C for 2 hours. After the reaction was completed, the conversion rate and optical purity of the reaction product (R)-3-chloro-1,2-propanediol were analyzed by the same method as in Example 9. As a result, the conversion rate was 100%, and the optical purity was 97.4%ee.

(Example 15) Method for synthesizing (R)-3-chloro-1,2-propanediol

Recombinant Escherichia coli HB101 (pNTS1G) Accession No. FERM BP-5835 were inoculated into 50 ml of sterilized 2×YT medium (16 g of tryptone, 10 g of yeast extract, 5 g of sodium chloride, and 1L of water, pH 7.0 before sterillization) in a 500 ml Sakaguchi flask, and cultured with shaking at 37°C for 18 hours. Then, 10 mg of 1-chloro-3-hydroxyacetone, 70 µg of $NAD^+$, and 25 mg of glucose were added to 1 ml of the resultant culture broth, and the mixture was then stirred at 30°C for 2 hours. After the reaction was completed, the conversion rate and optical purity of the reaction product (R)-3-chloro-1,2-propanediol were analyzed by the same method as in Example 9. As a result, the conversion rate was 96.4%, and the optical purity was 98.6%ee.

(Example 16) Method for synthesizing (S)-3,3-dichloro-1,2-propanediol

Recombinant Escherichia coli HB101 (pNTSGG1) Accession No. FERM P-18449 were inoculated into 50 ml of sterilized 2×YT medium (16 g of tryptone, 10 g of yeast extract, 5 g of sodium chloride, and 1L of water, pH 7.0 before sterillization) in a 500 ml Sakaguchi flask, and cultured with shaking at 37°C for 18 hours. Then, 10 mg of 2,5-dihydroxy-2,5-di(dichloromethyl)-1,4-dioxane, 0.6 mg of $NAD^+$, and 25 mg of glucose were added to 1 ml of the resultant culture broth, and the mixture was then stirred at 30°C for 2 hours. After the reaction was completed, the conversion rate and optical purity of the reaction product (S)-3,3-dichloro-1,2-propanediol were analyzed by the same method as in Example 9. As a result, the conversion rate was 100%, and the optical purity was 100%ee.

(Example 17) Method for synthesizing (S)-3,3-dichloro-1,2-propanediol

Recombinant Escherichia coli HB101 (pNTCRG) Accession No. FERM BP-6898 were inoculated into 50 ml of sterilized 2×YT medium (16 g of tryptone, 10 g of yeast extract, 5 g of sodium chloride, and 1L of water, pH 7.0 before sterillization) in a 500 ml Sakaguchi flask, and cultured with shaking at 37°C for 18 hours. Then, 10 mg of 2,5-dihydroxy-2,5-di(dichloromethyl)-1,4-dioxane, 70 µg of $NAD^+$, and 25 mg of glucose were added to 1 ml of the resultant culture broth, and the mixture was then stirred at 30°C for 2 hours. After the reaction was completed, the conversion rate and optical purity of the reaction product (S)-3,3-dichloro-1,2-propanediol were analyzed by the same method as in Example 9. As a result, the conversion rate was 100%, and the optical purity was 94.6%ee.

(Example 18) Method for synthesizing (R)-3,3-dichloro-1,2-propanediol

Recombinant Escherichia coli HB101 (pNTFPG) Accession No. FERM BP-7117 were inoculated into 50 ml of sterilized 2×YT medium (16 g of tryptone, 10 g of yeast extract, 5 g of sodium chloride, and 1L of water, pH 7.0 before sterillization) in a 500 ml Sakaguchi flask, and cultured with shaking at 37°C for 18 hours. Then, 10 mg of 2,5-dihydroxy-2,5-di(dichloromethyl)-1,4-dioxane, 70 µg of $NAD^+$, and 25 mg of glucose were added to 1 ml of the resultant culture broth, and the mixture was then stirred at 30°C for 2 hours. After the reaction was completed, the conversion rate and optical purity of the reaction product (R)-3,3-dichloro-1,2-propanediol were analyzed by the same

method as in Example 9. As a result, the conversion rate was 100%, and the optical purity was 91%ee.

(Example 19) Method for synthesizing (R)-3,3-dichloro-1,2-propanediol

Recombinant Escherichia coli HB101 (pNTS1G) Accession No. FERM BP-5835 were inoculated into 50 ml of sterilized 2×YT medium (16 g of tryptone, 10 g of yeast extract, 5 g of sodium chloride, and 1L of water, pH 7.0 before sterilization) in a 500 ml Sakaguchi flask, and cultured with shaking at 37°C for 18 hours. Then, 10 mg of 2,5-dihydroxy-2,5-di(dichloromethyl)-1,4-dioxane, 70 μg of $NAD^+$, and 25 mg of glucose were added to 1 ml of the resultant culture broth, and the mixture was then stirred at 30°C for 2 hours. After the reaction was completed, the conversion rate and optical purity of the reaction product (R)-3,3-dichloro-1,2-propanediol were analyzed by the same method as in Example 9. As a result, the conversion rate was 96.4%, and the optical purity was 98.6%ee.

Industrial Applicability

The method of the present invention is capable of simply producing optically active halopropanediol derivatives useful as pharmaceutical intermediates from inexpensive raw materials.

## Claims

1. A dihalohydroxyacetone derivative represented by formula (9) or (10) :

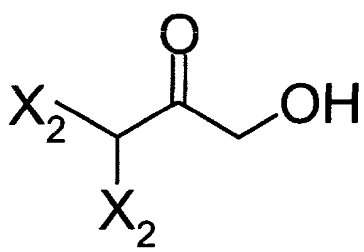

(9)

(wherein $X_2$ represents a halogen atom);

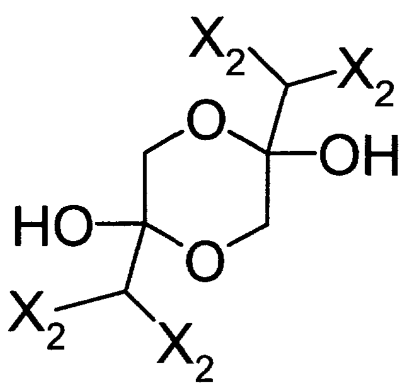

(10)

(wherein $X_2$ is the same as the above).

2. The compound according to claim 1, wherein $X_2$ is a chlorine atom.

3. A method for producing a di- or tri-halohydroxyacetone derivative represented by formula (3) or (4) :

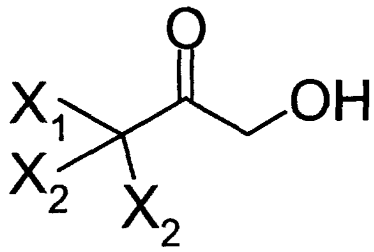

(3)

(wherein $X_1$ represents a hydrogen atom or a halogen atom, and $X_2$ represents a halogen atom);

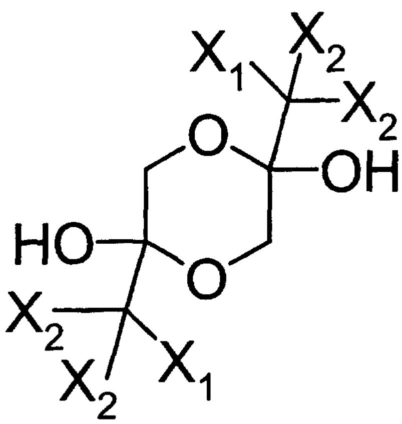

(4)

(wherein $X_1$ and $X_2$ are the same as the above), the method comprising reacting a propargyl alcohol derivative represented by formula (1);

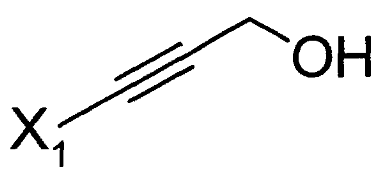

(1)

(wherein $X_1$ is the same as the above) and a hypohalogenous acid represented by formula (2);

$$X_2OH \quad (2)$$

(wherein $X_2$ is the same as the above).

**4.** The method according to claim 3, wherein in each of formulae (1), (3), and (4), $X_1$ is a hydrogen atom.

**5.** The method according to claim 3 or 4, wherein in each of formulae (2), (3), and (4), $X_2$ is a chlorine atom.

**6.** The method according to any one of claims 3 to 5, wherein, a sodium hypochlorite solution or chlorine is used as an agent for preparing the hypohalogenous acid represented by formula (2).

**7.** A method for producing a monohalohydroxyacetone derivative represented by formula (5) or (6):

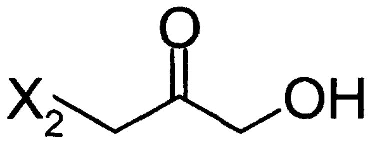

(5)

(wherein $X_2$ represents a halogen atom);

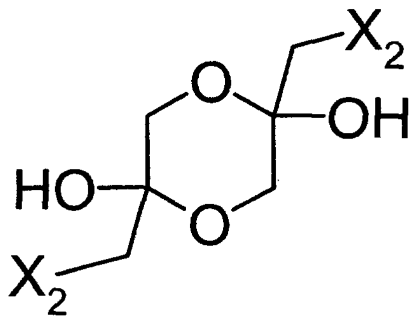

(6)

(wherein $X_2$ is the same as the above), the method comprising hydrogenating a di- or tri-halohydroxyacetone derivative represented by formula (3) or (4) :

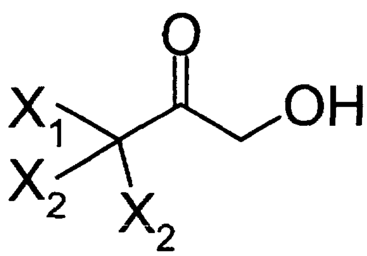

(3)

(wherein $X_1$ represents a hydrogen atom or a halogen atom, and $X_2$ represents a halogen atom);

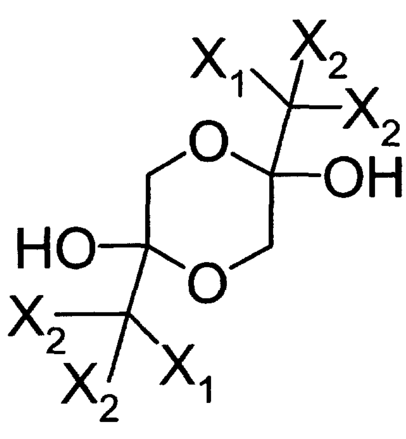

(4)

(wherein $X_1$ and $X_2$ are the same as the above)
in a solvent in the presence of a transition metal catalyst.

**8.** The method according to claim 7, wherein in each of formulae (3) and (4), $X_1$ is a hydrogen atom.

**9.** The method according to claim 7 or 8, wherein in each of formulae (3), (4), (5), and (6), $X_2$ is a chlorine atom.

**10.** The method according to any one of claims 7 to 9, wherein the transition metal catalyst is a platinum catalyst, a rhodium catalyst, a palladium catalyst or a nickel catalyst.

**11.** The method according to claim 10, wherein the transition metal catalyst comprises a metal of platinum, rhodium, palladium or nickel, an alloy of the metal, or a chloride, a bromide, an iodide, a nitrate, a sulfate, a phosphate, an oxide, a sulfide, a boride, a hydroxide, a cyanide, an acetylacetonate, an acetate, or a trifluoroacetate of said metal or alloy.

**12.** The method according to claim 10, wherein the transition metal catalyst comprises platinum oxide, palladium oxide, platinum black, palladium black, Raney nickel, or nickel boride.

**13.** The method according to claim 10, wherein the transition metal catalyst comprises a metal of platinum, rhodium or palladium, an alloy of the metal, a sulfide or a hydroxide of said metal or alloy, which is carried by carbon, alumina, silica-alumina, silica, barium carbonate, barium sulfate, calcium carbonate, titanium oxide, zirconium oxide, zeolite, or asbesto.

**14.** The method according to claim 13, wherein the transition metal catalyst comprises palladium-carbon, rhodium-carbon, platinum-carbon, palladium-alumina, platinum-alumina, palladium-calcium carbonate, platinum-calcium carbonate, palladium-barium sulfate, or platinum-barium sulfate.

**15.** The method according to any one of claims 7 to 14, wherein the reaction solvent is a protic solvent.

**16.** The method according to claim 15, wherein the protic solvent is an alcohol solvent or water.

**17.** A method for producing an optically active halopropanediol derivative represented by formula (7) or (8) ;

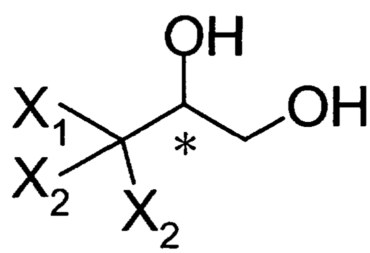

(7)

(wherein $X_1$ represents a hydrogen atom or a halogen atom, $X_2$ represents a halogen atom, and * represents an asymmetric carbon atom);

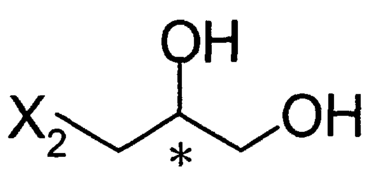

(8)

(wherein $X_2$ and * are the same as the above), the method comprising stereoselectively reducing a halohydroxyacetone derivative represented by any one of formulae (3), (4), (5), and (6) with an enzyme source having an ability to stereoselectively reduce the carbonyl group of a di- or tri-halohydroxyacetone derivative represented by formula (3) or (4), or a monohalohydroxyacetone derivative represented by formula (5) or (6):

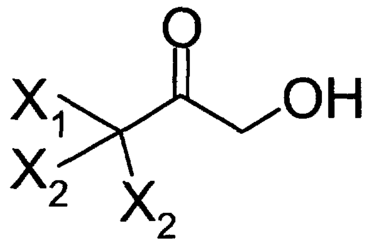

(3)

(wherein $X_1$ and $X_2$ are the same as the above);

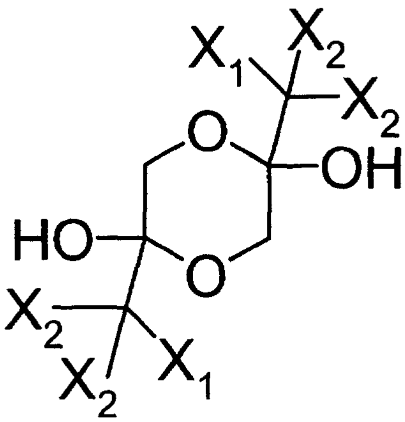

(4)

(wherein $X_1$ and $X_2$ the same as the above);

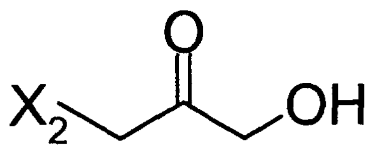

(5)

(wherein $X_2$ is the same as the above);

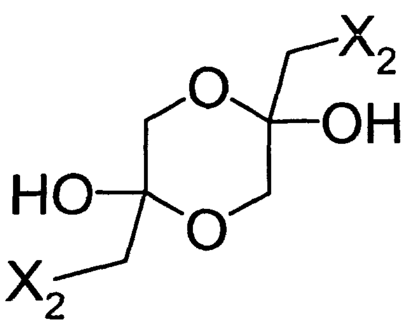

(6)

(wherein $X_2$ is the same as the above).

**18.** The method according to claim 17, wherein the enzyme source having the ability to stereoselectively reduce the carbonyl group of the halohydroxyacetone derivative is cells, a culture broth or a treated product of at least one microorganism belonging to a genus selected from the group consisting of Ambrosiozyma, Brettanomyces, Candida, Debaryomyces, Dipodascus, Geotrichum, Galactomyces, Issatchenkia, Kluyveromyces, Lodderomyces, Metschnikowia, Ogataea, Pichia, Rhodosporidium, Rhodotorula, Saccharomycopsis, Stephanoascus, Torulaspora, Trigonopsis, Trichosporon, Yamadazyma, Yarrowia, Cellulomonas, Enterobacter, Jensenia, Klebsiella, Microbacterium, Morganella, Nocardia, Rhodococcus, Serratia, Absidia, Acrotheca, Acremonium, Aegerita, Aspergillus, Auxarthron, Byssochlamys, Chaetomidium, Cladosporium, Corynascus, Corynespora, Cryptophiale, Coriolus, Dendryphiella, Fistulina, Fusarium, Gibberella, Macrophoma, Mortierella, Mucor, Neocosmospora, Paecilomyces, Panus, Penicillium, Pleurotus, Plectosphaerella, Rhizopus, Sclerotinia, Sclerotium, Scopulariopsis, Schizophyllum, Sphaerodes, Tyromyces, Verticillium, Wardomyces and Streptomyces, and/or an enzyme derived from the microorganism.

**19.** The method according to claim 17, wherein the enzyme source having the ability to stereoselectively reduce the carbonyl group of the halohydroxyacetone derivative is an enzyme source having a (R)-selectively reducing ability and is cells, a culture broth or a treated product of at least one microorganism belonging to a genus selected from the group consisting of Brettanomyces, Candida, Debaryomyces, Dipodascus, Geotrichum, Galactomyces, Issatchenkia, Kluyveromyces, Lodderomyces, Ogataea, Pichia, Rhodosporidium, Rhodotorula, Saccharomycopsis, Stephanoascus, Torulaspora, Trigonopsis, Trichosporon, Yamadazyma, Yarrowia, Enterobacter, Klebsiella, Microbacterium, Absidia, Acrotheca, Acremonium, Aegerita, Aspergillus, Chaetomidium, Cladosporium, Corynespora, Cryptophiale, Fusarium, Gibberella, Macrophoma, Mucor, Neocosmospora, Paecilomyces, Penicillium, Pleurotus, Plectosphaerella, Rhizopus, Sclerotinia, Sclerotium, Sphaerodes, Tyromyces, Verticillium, and Streptomyces, and/or an enzyme derived from the microorganism.

**20.** The method according to claim 19, wherein the enzyme source having the ability to (R)-selectively reduce the carbonyl group of the halohydroxyacetone derivative is cells, a culture broth or a treated product of at least one microorganism selected from the group consisting of Brettanomyces anomalus, Candida etchellsii, Candida gropengiesseri, Candida magnoliae, Candida maris, Debaryomyces hansenii, Debaryomyces robertsiae, Dipodascus ovetensis, Dipodascus tetrasperma, Geotrichum fermentans, Galactomyces reessii, Issatchenkia terricola, Kluyveromyces thermotolerans, Lodderomyces elongisporus, Ogataea minuta, Pichia bovis, Pichia anomala, Pichia silvicola, Rhodosporidium toruloides, Rhodotorula aurantiaca, Rhodotorula araucariae, Rhodotorula lactosa, Saccharomycopsis selenospora, Saccharomycopsis vini, Stephanoascus ciferrii, Torulaspora delbrueckii, Trigonopsis variabilis, Trichosporon asteroides, Yamadazyma haplophila, Yarrowia lipolytica, Enterobacter aerogenes, Klebsiella planticola, Klebsiella pneumoniae, Microbacterium arborescens, Absidia coerulea, Acrotheca cerophila, Acremonium butyri, Aegerita candida, Aspergillus versicolor, Chaetomidium fimeti, Cladosporium resinae, Corynespora cassiicola, Cryptophiale guadalcanalense, Fusarium oxysporum, Fusarium anguioides, Gibberella fujikuroi, Macrophoma commelinae, Mucor tuberculisporus, Mucor inaequisporus, Neocosmospora vasinfecta, Paecilomyces lilacinus, Penicillium janthinellum, Pleurotus ostreatus, Plectosphaerella cucumerina, Rhizopus niveus, Rhizopus oryzae, Rhizopus stolonifer, Sclerotinia sclerotiorum, Sclerotium delphinii, Sphaerodes fimicola, Tyromyces palustris, Verticillium niveostratosum, Streptomyces coelescens, and Streptomyces lividans, and/or an enzyme derived from the microorganism.

**21.** The method according to claim 20, wherein the enzyme source having the ability to (R)-selectively reduce the carbonyl group of the halohydroxyacetone derivative is Escherichia coli HB101 (pNTS1G) Accession No. FERM BP-5835 or Escherichia coli HB101 (pNTFPG) Accession No. FERM BP-7117.

**22.** The method according to claim 17, wherein the enzyme source having the ability to stereoselectively reduce the carbonyl group of the halohydroxyacetone derivative is an enzyme source having a (S)-selectively reducing ability and is cells, a culture broth or a treated product of at least one microorganism belonging to a genus selected from the group consisting of Ambrosiozyma, Candida, Kluyveromyces, Metschnikowia, Yamadazyma, Cellulomonas, Jensenia, Microbacterium, Morganella, Nocardia, Rhodococcus, Serratia, Aspergillus, Auxarthron, Byssochlamys, Corynascus, Coriolus, Dendryphiella, Fistulina, Mortierella, Panus, Scopulariopsis, Schizophyllum, Wardomyces and Streptomyces, and/or an enzyme derived from the microorganism.

**23.** The method according to claim 22, wherein the enzyme source having the ability to (S)-selectively reduce the carbonyl group of the halohydroxyacetone derivative is cells, a culture broth or a treated product of at least one microorganism selected from the group consisting of Ambrosiozyma philentoma, Candida magnoliae, Candida tenuis, Kluyveromyces polysporus, Metschnikowia bicuspidata, Yamadazyma stipitis, Cellulomonas sp., Cellulomonas uda, Cellulomonas fimi, Jensenia canicruria, Microbacterium arborescens, Morganella morganii, Nocardia globerula, Rhodococcus erythropolis, Rhodococcus equi, Rhodococcus rhodochrous, Serratia marcescens, Aspergillus sydowii, Auxarthron thaxteri, Byssochlamys fulva, Corynascus sepedonium, Coriolus consors, Dendryphiella salina, Fistulina hepatica, Mortierella vinacea, Panus lacomtei, Scopulariopsis brevicaulis, Schizophyllum commune, Wardomyces anomalus, Streptomyces cacaoi, Streptomyces celluloflavus, Streptomyces diastatochromogenes, Streptomyces hydrogenans, Streptomyces achromogenes, and Streptomyces salmonis, and/or an enzyme derived from the microorganism.

**24.** The method according to claim 23, wherein the enzyme source having the (S)-selectively reduce the carbonyl group of the halohydroxyacetone derivative is Escherichia coli HB101 (pNTCRG) Accession No. FERM BP-6898 or Escherichia coli HB101 (pNTSGG1) Accession No. FERM P-18449.

**25.** The method according to claim 17, wherein the enzyme source having the ability to stereoselectively reduce the carbonyl group of the halohydroxyacetone derivative is an enzyme source having a (S)-selectively reducing ability and comprises glycerol dehydrogenase.

**26.** The method according to claim 25, wherein the glycerol dehydrogenase is an enzyme derived from cells, a culture broth or a treated product of at least one microorganism belonging to a genus selected from the group consisting of Cellulomonas and Serratia, and/or enzyme derived from the microorganism.

**27.** A method for isolating and purifying 2,5-dichloromethyl-2,5-dihydroxy-1,4-dioxane, the method comprising removing an impurity from 2,5-dichloromethyl-2,5-dihydroxy-1,4-dioxane represented by formula (11) by using an organic solvent to obtain the compound represented by formula (11) as crystals:

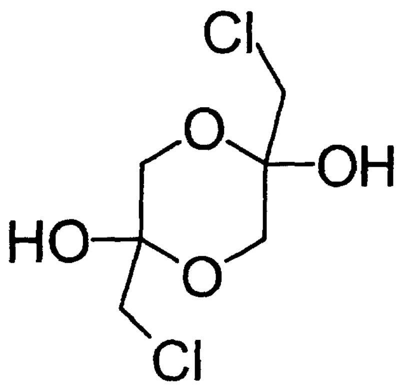

(11)

**28.** The method according to claim 27, wherein the impurity contained in 2,5-dichloromethyl-2,5-dihydroxy-1,4-dioxane represented by formula (11) is acetol, 1,1-dichloro-3-hydroxyacetone, 2,5-di(dichloromethyl)-2,5-dihydroxy-1,4-dioxane, 1,3-dichloroacetone or 1,3-dihydroxyacetone.

**29.** The method according to claim 27 or 28, wherein the organic solvent is at least one selected from the group consisting of aromatic hydrocarbon solvents, ester solvents, ether solvents, ketone solvents, nitrile solvents, halogenated solvents, and alcohol solvents.

**30.** The method according to any one of claims 27 to 29, wherein the organic solvent is at least one selected from the group consisting of benzene, toluene, o-xylene, m-xylene, p-xylene, ethylbenzene, cumene, n-butylbenzene, 1,3,5-mesitylene, ethyl formate, methyl acetate, ethyl acetate, n-propyl acetate, n-butyl acetate, tert-butyl acetate, methyl propionate, ethyl propionate, γ-butyrolactone, tert-butyl methyl ether, diethyl ether, diisopropyl ether, di-n-butyl ether, dimethoxyethane, diethylene glycol dimethyl ether, tetrahydrofuran, 1,4-dioxane, anisole, acetone, methyl ethyl ketone, diethyl ketone, cyclopentanone, cyclohexanone, acetonitrile, propionitrile, methylene chloride, chloroform, carbon tetrachloride, 1,2-dichloroethane, 1,1,1-trichloroethane, chlorobenzene, methanol, ethanol, n-propanol, isopropanol and n-butanol.

**31.** The method according to any one of claims 27 to 30, wherein an auxiliary solvent is also used.

**32.** The method according to claim 31, wherein the auxiliary solvent is an aliphatic hydrocarbon solvent.

**33.** The method according to claim 32, wherein the aliphatic hydrocarbon solvent is at least one selected from the

group consisting of pentane, petroleum ether, neopentane, hexane, cyclohexane, methyl cyclohexane, heptane, cycloheptane, octane, isooctane, nonane and decane.

**34.** The method according to any one of claims 31 to 33, wherein the auxiliary solvent is used in a ratio (organic solvent/auxiliary solvent) by volume of the organic solvent to the auxiliary solvent of 10 or less at the end of a crystallization operation.

**35.** The method according to any one of claims 27 to 34, wherein 2,5-dichloromethyl-2,5-dihydroxy-1,4-dioxane represented by formula (11), produced by the method according to any one of claims 7 to 16 is used.

## INTERNATIONAL SEARCH REPORT

International application No.
PCT/JP02/08624

**A. CLASSIFICATION OF SUBJECT MATTER**

Int.Cl[7] C07C49/173, 45/26, 45/45, 45/65, C07D319/12, C12P7/18

According to International Patent Classification (IPC) or to both national classification and IPC

**B. FIELDS SEARCHED**

Minimum documentation searched (classification system followed by classification symbols)

Int.Cl[7] C07C49/173, 45/26, 45/45, 45/65, C07D319/12, C12P7/18

Documentation searched other than minimum documentation to the extent that such documents are included in the fields searched

Electronic data base consulted during the international search (name of data base and, where practicable, search terms used)

CA(STN), REGISTRY(STN), CASREACT(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | MEISTER H., "Darstellung und Hydrolyse von 4-Halogenmethylen-1.3-dioxolanen", Liebigs Ann. Chem., 1969, Vol.724, pages 128 to 136 | 27-34<br>17-26<br>1-16,35 |
| Y<br>A | JP 1-320988 A (Kaneka Corp.), 27 December, 1989 (27.12.89), Claims (Family: none) | 17-26<br>1-16,27-35 |
| Y<br>A | JP 11-103878 A (Mitsubishi Chemical Corp.), 20 April, 1999 (20.04.99), Claims (Family: none) | 17-26<br>1-16,27-35 |
| A | ANDO T. et al., "Total Syntheses of Carbohydrates. I. Dihydroxyacetone and DL-Erythrulose", Bull.Chem. Soc.Jpn., 1972, Vol.45, pages 2611 to 2615 | 1-35 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 November, 2002 (22.11.02) | 10 December, 2002 (10.12.02) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/08624

C (Continuation). DOCUMENTS CONSIDERED TO BE RELEVANT

| Category* | Citation of document, with indication, where appropriate, of the relevant passages | Relevant to claim No. |
|---|---|---|
| A | VERARDO G., "Addition of Methylene Group Across the Ester Carbonyl Function", Tetrahedron Letters, 1987, Vol.28, No.26, pages 3011 to 3012 | 1-35 |
| A | US 4008279 A (Celanese Corp.), 15 February, 1977 (15.02.77), & JP 51-41314 A & DE 2536395 A1 & FR 2281915 A1 | 1-35 |

Form PCT/ISA/210 (continuation of second sheet) (July 1998)